# EUROPEAN PATENT APPLICATION

(11) **EP 0 950 715 A2**
(43) Date of publication of application: **20.10.1999**
(21) Application number: 99106676.2
(22) Date of filing: 01.04.1999
(51) Int. Cl.: C12P 17/12

(54) **Enzymatic production of vitamin B6**

(30) Priority: 15.04.1998 EP 98106812
(71) Applicant: F. HOFFMANN-LA ROCHE AG, 4070 Basel (CH)
(72) Inventor: Hoshino, Tatsuo, Kamakura-shi, Kanagawa-ken (JP); Tazoe, Masaaki, Yokohama-shi, Kanagawa-ken (JP)

(57) **Abstract**

A process for an enzymatic production of vitamin B₆ which comprises incubating 1-deoxy-D-threo-pentulose and 4-hydroxy-L-threonine with an enzyme reaction system prepared from cells of microorganism belonging to genus *Rhizobium, Sinorhizobium, Flavobacterium, Chryseobacterium, Lactobacillus, Arthrobacter, Bacillus, Klebsiella, Escherichia, Pseudomonas, Stenotrophomonas, Enterobacter, Serratia, Corynebacterium, Brevibacterium, Exiguobacterium, Saccharomyces, Yamadazma, Pichia* and *Candida*, in the presence of NADP⁺, NAD⁺, ATP. Manganese and magnesium ions stimulate the above reaction. This process affords high yields of vitamin B₆, a vitamin essential for the nutrition of animals, plants and microorganisms, and useful as a medicine or food additive.

## Description

This invention relates to a process for the enzymatic production of vitamin B₆ from 1-deoxy-D-threo-pentulose (referred to hereinafter as DTP) and 4-hydroxy-L-threonine (HT). The expression "vitamin B₆" as used in the present invention includes pyridoxol, pyridoxal and pyridoxamine.

Vitamin B₆ is one of the essential vitamins for the nutrition of animals, plants and microorganisms, and is also very important as a medicine or food additive for humans. The object of the present invention is to provide a highly efficient process for the enzymatic production of vitamin B₆ from DTP and HT.

There are many studies on the fermentative production of vitamin B6. Various microorganisms belonging to the genera *Saccharomyces* [G. H. Scherr and M. E. Rafelson, J. Appl. Bacteriol. 25, 187-194 (1962)], *Pichia* [N. Nishino, K. Fujii, and T. Kamikubo, Agric. Biol. Chem. 37, 553-559 (1973)], *Klebsiella* [R. Suzue and Y. Haruna, J. Vitaminol. 16, 154-159 (1970)], *Achromobacter* [M. Ishida and K. Shimura, Agric. Biol. Chem. 34, 327-334 (1970)], *Bacillus* [W. Pflug and F. Lingens, Hoppe-Seyler's Z. Physiol. Chem. 359, 559-570 (1978)] and *Flavobacterium* [Y. Tani, T. Nakamatsu, T. Izumi and K. Ogata, Agric. Biol. Chem. 36, 189-197 (1972)] are known to produce vitamin B₆. But no commercially attractive fermentation process for the production of vitamin B₆ has become known so far. More recently, there has been described in the European Patent Publication 0 765 938 A2 a process for the fermentative production of vitamin B₆ by cultivating a microorganism belonging to the genus *Rhizobium* capable of producing said vitamin in a culture medium under aerobic conditions; the culture medium may contain, apart from assimilable carbon and digestible nitrogen sources, inorganic salts and other nutrients, further substances which improve the vitamin B₆ titer, such as DTP and HT. Although this more recently known process produces vitamin B₆ in high yield, there is room for improvement of its efficiency.

The present invention makes it possible to produce vitamin B₆ from DTP and HT in higher efficiency than hitherto. It has been found that the cell-free extract prepared from the cells of a microorganism such as of the genus *Rhizobium*, *Sinorhizobium*, *Flavobacterium*, *Chryseobacterium*, *Lactobacillus*, *Arthrobacier*, *Bacillus*, *Klebsiella*, *Escherichia*, *Pseudomonas*, *Stenotrophomonas*, *Enterobacter*, *Serratia*, *Corynebacterium*, *Brevibacterium*, *Exiguobacterium*, *Saccharomyces*, *Yamadazma*, *Pichia* or *Candida* is capable of producing vitamin B₆ from DTP and HT in the presence of nicotinamide adenine dinucleotide phosphate (NADP⁺), nicotinamide adenine dinucleotide (NAD⁺) and adenosine triphosphate (ATP).

The present invention is thus concerned with a process for the enzymatic production of vitamin B₆ from DTP and HT which comprises contacting DTP and HT with an enzyme reaction system prepared from cells of a microorganism capable of producing vitamin B₆ from DTP and HT, said contacting occurring in the presence of NADP⁺, NAD⁺ and ATP. The present invention is further concerned with a process for the enzymatic production of vitamin B₆ which comprises contacting DTP and HT with an enzyme reaction system containing the cell-free extract derived from a microorganism capable of producing vitamin B₆, such as one belonging to the genus *Rhizobium*, *Sinorhizobium*, *Flavobacterium*, *Chryseobacterium*, *Lactobacillus, Arthrobacter, Bacillus, Klebsiella, Escherichia, Pseudomonas, Strenotrophomonas, Enterobacter, Serratia, Corynebacterium, Brevibacterium, Exiguobacterium, Saccharomyces, Yamadazma, Pichia* or *Candida*, in the presence of NADP⁺, NAD⁺ and ATP.

The content of vitamin B₆ in a reaction mixture can be determined by a bioassay with *Saccharomyces carlsbergensis* ATCC 9080 according to the method of D. R. Osborne and P. Voogt. [The Analysis of Nutrients in Foods, Academic Press, London, 224-227 (1978)].

For carrying out the process of the present invention, cells of the microorganism, e.g. one belonging to the genus *Rhizobium, Sinorhizobium, Flavobacterium, Chryseobacterium, Lactobacillus, Arthrobacter, Bacillus, Klebsiella, Escherichia, Pseudomonas, Stenotrophomonas, Enterobacter, Serratia, Corynebacterium, Brevibacterium, Exiguobacterium, Saccharomyces, Yamadazma, Pichia* or *Candida*, can be produced by cultivating said microorganism in a medium containing assimilable carbon sources, digestible nitrogen sources, inorganic salts and other nutrients necessary for the growth of the microorganism. As the carbon sources, for example, glucose, fructose, lactose, galactose, sucrose, maltose, starch, dextrin and glycerol may be employed. As the nitrogen sources, for example, peptone, yeast extract, soybean powder, corn steep liquor, meat extract, ammonium sulfate, ammonium nitrate, urea, and mixtures thereof may be employed. Further, as the inorganic salts sulfates, hydrochlorides or phosphates of calcium, magnesium, zinc, manganese, cobalt and iron may be employed. And, if necessary, conventional nutrient factors or an antifoaming agent, such as animal oil, vegetable oil or mineral oil, can also be included in the culture medium. The pH of the culture medium may be from about 5 to about 9, preferably from about 6 to about 8. The temperature range for the cultivation is suitably from about 10°C to about 45°C, preferably from about 25°C to about 40°C. The cultivation time is normally from about 1 to about 5 days, preferably from about 1 to about 3 days. Aeration and agitation during the cultivation usually give favorable results.

The microorganisms which can be used in the process of the present invention include all the strains belonging to the genera *Rhizobium, Sinorhizobium, Flavobacterium, Chryseobacterium, Lactobacillus, Arthrobacter, Bacillus, Klebsiella, Escherichia, Pseudomonas, Stenotrophomonas, Enterobacter, Serratia, Corynebacterium, Brevibacterium, Exiguobacterium, Saccharomyces, Yamadazma, Pichia* and *Candida.* Such microorganisms are available from a public depository (culture collection) to anyone upon request, such as the Institute of Fermentation, Osaka, Japan (IFO): examples of such deposited strains are *Rhizobium meliloti* (also known as *Sinorhizobium meliloti*) IFO 14782 (DSM No. 10226), *Flavobacterium indologenes* (also known as *Chryseobacterium indologene*s) IFO 14944, *Lactobacillus brevis* IFO 13110, *Arthrobacter nicotianae* IFO 14234, *Bacillus subtilis* IFO 3007, *Klebsiella planticola* IFO 3317, *Escherichia coli* IFO 13168, *Pseudomonas putida* IFO 3738, *Stenotrophomonas maltophilia* (also known as *Pseudomonas maltophilia* or Xanthomonas *maltophilia*) IFO 12692, *Enterobacter cloacae* IFO 3320, *Serratia marcescens* IFO 12648, *Corynebacterium ammoniagenes* (also known as *Brevibacterium ammoniagenes*) IFO 12612, *Corynebacterium glutamicum* (also known as *Brevibacterium glutamicum*) IFO 12168, *Exiguobacterium acetylicum* (also known as *Brevibacterium acetylicum*) IFO 12146, *Pichia guilliermondii* (also known as *Yamadazyma guilliermondii*) IFO 10106, *Saccharomyces cerevisiae* IFO 0304 and IFO 0306 and *Candida tropicalis* IFO 0199 and IFO 0587. Among these microorganisms, the following are preferably used in the present invention: *Rhizobium meliloti* IFO 14782 (DSM No. 10226), *Flavobacterium indologenes* IFO 14944, *Bacillus subtilis* IFO 3007, *Escherichia coli* IFO 13168, *Serratia marcescens* IFO 12648, *Corynebacterium ammoniagenes* IFO 12612, *Corynebacterium glutamicum* IFO 12168, *Pichia guilliermondii* IFO 10106 and *Saccharomyces cerevisiae* IFO 0306.

For preparation of the cell-free extract from the cells obtained by cultivation, general methods such as sonication and cell breakage in the presence of glass beads or by the French press homogenizer can be applied. If desired, treatment with a lytic enzyme such as lysozyme or zymolase at 15°C to 45°C, preferably at 20°C to 40°C for 1 to 3 hours can be also applied before the disruption in the above-mentioned way. For example, after centrifugation of the culture broth, the resulting cells are washed with saline and suspended in a buffer such as Tris-HCl (pH 7.5) buffer containing sucrose, dithiothreitol (DTT) and phenylmethylsulfonyl fluoride (PMSF) as general stabilizers of enzymes. After cell breakage, the resulting solution is centrifuged to separate the cell debris, and its supernatant can be used as the cell-free extract.

The enzyme reaction system contains the cell-free extract as prepared above or those partially purified by general methods for purification of enzymes such as ammonium sulfate precipitation or gel filtration chromatography. Alternatively, the resting cells or the growing cells of the microorganism can also be used. In addition to the cell-free extract, DTP and HT as substrates and also NADP⁺, NAD⁺ and ATP as cofactors are added to the reaction system. The amount of DTP, HT, NADP⁺, NAD⁺ and ATP to be added to the system can be varied depending on the reaction system employed. But, in general, the concentrations of DTP, HT, NADP⁺, NAD⁺ and ATP in the enzyme reaction system are 0.1mM or more, preferably from 1mM to 10mM for DTP and HT, preferably from 0.05 mM to 5 mM, more preferably from 0.2 mM to 0.4 mM, for NADP⁺ and NAD⁺, and preferably from 1 mM to 20 mM, more preferably from 3 mM to 7 mM, for ATP. The addition of manganese ions or magnesium ions to the enzyme reaction system stimulates the reaction, and the addition of both such ions produces even more preferable results. As salts giving rise to such ions, for example, the hydrochlorides, sulfates, nitrates or phosphates of manganese and magnesium can be employed. The amount of manganese ions and magnesium ions to be added can also be varied depending on the reaction system employed. But, in general, the concentrations of manganese ions and magnesium ions are in the case of manganese ions from 0.1 mM to 100 mM, preferably from 5 mM to 10 mM, and in the case of magnesium ions from 3 mM to 300 mM, preferably from 20 mM to 50 mM.

For initiating the enzyme reaction, a buffer solution which has no influence on vitamin B₆ production from DTP and HT can be used. Tris-HCl buffer is preferably used for this purpose. The enzyme reaction is suitably effected in a pH range from 6.0 to 8.5, more preferably in the range from 7.0 to 8.0. The reaction temperature is suitably from 15°C to 45°C, preferably from 20°C to 40°C. The incubation period may be varied depending on the reaction conditions, but is generally from 30 minutes to 5 hours, more preferably from 2 hours to 4 hours.

Vitamin B₆ produced from DTP and HT under the conditions as described above can easily be recovered as follows. For example, after the reaction, proteins in the reaction mixture are precipitated by denaturation with heat, acid, alkali or organic solvent and removed by centrifugation. For this purpose a process generally used for extracting a certain product from the above supernatant may be employed which is applicable to the various properties of vitamin B₆. Thus, for example, vitamin B₆ in the supernatant is purified with an ion exchange resin. The desired product is further recrystallized from a mixture of alcohol and water.

The present invention will be illustrated in more detail by the following Examples; however, it should be understood that the present invention is not limited to these particular Examples.

### Example 1

### Preparation of cell-free extract

*Rhizobium meliloti* IFO 14782 (DSM No. 10226) was cultured in a seed medium containing 1% glucose, 0.5% polypeptone (Nippon Seiyaku Co., Japan), 0.2% yeast extract (Difco), 0.05% MgSO₄·7H₂O, 0.001% MnSO₄·5H₂O, and 0.001% FeSO₄·7H₂O at 28°C for 17 hours. The seed culture was transferred into a 500 ml flask containing 200 ml of a fermentation medium comprising 4% glucose, 2% polypeptone, 0.2% yeast extract, 0.05% MgSO₄·7H₂O, 0.05% MnSO₄·5H₂O, 0.001% FeSO₄·7H₂O, and one drop of antifoam CA-115 (Nippon Yushi Co., Japan) and then the flask was shaken on a flask shaker at 28°C. After cultivation for 72 hours, cells were harvested from 400 ml of the culture broth by centrifugation at 10,400 x g for 10 minutes and washed twice with 0.85% NaCl solution and washed once with 10 mM Tris-HCl (pH 7.5) buffer containing 15% sucrose, 0.1 mM PMSF and 1 mM DTT and stored at -30°C until use for preparation of the cell-free extract.

The following operation was all performed in ice water or at 4°C. The cells stored at - 30°C were thawed and suspended in 5 ml of 10 mM Tris-HCl (pH 7.5) buffer containing 15% sucrose, 0.1 mM PMSF and 1 mM DTT. The cell suspension was passed through a French press homogenizer (Ohtake Works Co,. Ltd.) at 200 kg/cm². The resulting homogenate was centrifuged at 34,800 x g for 30 minutes to remove cell debris. Ten milliliters of the supernatant were dialyzed overnight against 1 liter of 80% ammonium sulfate solution containing 15% sucrose, 0.1 mM PMSF and 1 mM DTT, and the precipitate was collected by centrifugation at 34,800 x g for 30 minutes. The precipitate was dissolved in 10 milliliters of 10 mM Tris-HCl buffer (pH 7.5) containing 15% sucrose and 0.1 mM PMSF, dialyzed overnight against the same buffer, the dialyzed solution was stored at -30°C until use for the enzyme reaction. The protein content in the cell-free extract was determined by the Lowry method [Lowry et al., J. Biol. Chem. 193, 265 (1951)] to be 11.4 mg/ml.

### Example 2

### Enzymatic production of vitamin B6 from DTP and HT

The enzyme reaction was carried out by incubating tubes containing 500 µl of the reaction mixture listed in **Table 1** at 28°C. A complete reaction system contained 2.5 mM DTP, 2.5 mM HT, 0.38 mM NADP⁺, 0.38 mM NAD⁺, 5 mM ATP, 193.25 µl of the cell-free extract and 80 mM Tris-HCl buffer, pH 7.50. After incubation for 2 hours, the reaction was stopped by heating in a boiling water bath for 3 minutes, centrifuged at 10,000 x g for 10 minutes and then the supernatant was treated with phosphatase by incubating a tube containing 15 µl of the supernatant, 10 µl of 1 mg/ml acid phosphatase (Boehringer Mannheim GmbH, Germany) and 10 µl of 100 mM acetate buffer (pH 5.0) at 37°C for 30 minutes. After incubation, 1,800 µl of water were added to the tube and determined by the microbiological method using *Saccharomyces carlsbergensis* ATCC 9080 as described bellow. The standard solutions of pyridoxol (0-2 µg per milliliter) were diluted 1.21 x 10⁻² in distilled water. One hundred µl of the diluted standard solution or sample and 3 ml of the assay medium for vitamin B₆ (Nissui Co., Japan) containing *Saccharomyces carlsbergensis* ATCC 9080 were added to tubes in this order and incubated with an angle of 30° at 28°C. After incubation for 17 hours, the cell growth was stopped by adding 5 ml of 0.1 N hydrochloric acid, and then the absorbance of the samples was measured at 660 nm. The amount of vitamin B₆ in a sample was determined by comparing the turbidity of the sample with the standard growth curve of *Saccharomyces carsbergensis* ATCC 9080. As a result, 97 ng of vitamin B₆/ml/mg protein/hour were produced in the complete reaction system. On the other hand, no vitamin B₆ was produced in the reaction systems omitting one factor from the complete system. Furthermore, 119, 123 or 587 ng of vitamin B₆/ml/mg protein/hour were produced in the system supplemented by 8.4 mM MnCl₂, 32 mM MgCl₂ or both, respectively, to the complete system (**Table 1**). The results indicate that cell-free extract, NADP⁺, NAD⁺ and ATP are essential for the vitamin B₆ production from DTP and HT, and that MnCl₂ and MgCl₂ stimulate the production.

**Table 1**

| Enzymatic production of vitamin B₆ from DTP and HT | |
|---|---|
| Reaction mixture | Produced vitamin B₆ (ng/ml/mg protein/ hr) |
| Complete reaction system | 97 |
| Complete minus cell-free extract | 0 |
| Complete minus HT | 0 |
| Complete minus DTP | 0 |
| Complete minus NADP⁺ | 0 |
| Complete minus NAD⁺ | 0 |
| Complete minus ATP | 0 |
| Complete plus 8.4 mM MnCl₂ | 119 |
| Complete plus 32 mM MgCl₂ | 123 |
| Complete plus 8.4 mM MnCl₂ and 32 mM MgCl₂ | 587 |
| Complete: 2.5 mM DTP, 2.5 mM HT, 0.38 mM NADP⁺, 0.38 mM NAD⁺, 5 mM ATP, 193.25 µl cell-free extract and 80 mM Tris-HCl buffer, pH 7.50 | |

### Example 3

In a similar manner as described in Example 1 and 2, the vitamin B₆ production by the cell-free extracts of various kind of microorganisms was examined. A loopful of cells grown on the agar plate of each strain listed in **Table 2** was cultured in each seed medium at 28°C for 17 hours. Two milliliters of the seed culture were transferred into a 500 ml flask containing 100 ml of the bulk medium and one drop of antifoam, and then the flask was shaken on a flask shaker at 28°C. The compositions of seed and bulk media for cultivation of each strain listed on **Table 2** are summarized in **Table 3**.

**Table 2**

| Microorganism and their cultivation media | | |
|---|---|---|
| Microorganism | Media | |
| | Seed | Bulk |
| *Flavobacterium indologenes* IFO 14944 | SM2 | FM2 |
| *Lactobacillus brevis* IFO 13110 | SM1 | FM1 |
| *Arthrobacter nicotianae* IFO 14234 | SM2 | FM2 |
| *Bacillus subtilis* IFO 3007 | SM2 | FM2 |
| *Klebsiella planticola* IFO 3317 | SM2 | FM2 |
| *Escherichia coli* IFO 13168 | SM2 | FM2 |
| *Pseudomonas putida* IFO 3738 | SM2 | FM2 |
| *Stenotrophomonas maltophilia* IFO 12692 | SM2 | FM2 |
| *Enterobacter cloacae* IFO 3320 | SM2 | FM2 |
| *Serratia marcescens* IFO 12648 | SM2 | FM2 |
| *Corynebacterium ammoniagenes* IFO 12612 | #802 | #802 |
| *Corynebacterium glutamicum* IFO 12168 | #802 | #802 |
| *Exiguobacterium acetylicum* IFO 12146 | #802 | #802 |
| *Pichia guilliermondii* IFO 10106 | ME | ME |
| *Saccharomyces cerevisiae* IFO 0304 | ME | ME |
| *Saccharomyces cerevisiae* IFO 0306 | ME | ME |
| *Candida tropicalis* IFO 0199 | ME | ME |
| *Candida tropicalis* IFO 0587 | ME | ME |

**Table 3**

| Compositions of the media | | | | | | |
|---|---|---|---|---|---|---|
| Ingredient | SM1 | SM2 | #802 | ME | FM1 | FM2 |
| glucose | - | 1 | - | 1 | - | 2 |
| peptone (Nippon Seiyaku) | 0.5 | 0.5 | 1.0 | 0.5 | 2 | 2 |
| yeast extract (Difco) | 0.2 | 0.2 | 0.2 | 0.3 | 0.2 | 0.2 |
| malt extract (Difco) | - | - | - | 0.3 | - | - |
| MgSO₄·7H₂O | 0.05 | 0.05 | 0.1 | | 0.05 | 0.05 |
| MnSO₄·5H₂O | 0.001 | 0.001 | - | | 0.05 | 0.05 |
| FeSO₄·7H₂O | 0.001 | 0.001 | - | | 0.001 | 0.001 |

After cultivation for 24 hours, the cells of each strain were harvested from 400 ml of culture broth by centrifugation and washed twice with 0.85% NaCl solution and once with 10 mM Tris-HCl (pH 7.5) buffer containing 15% sucrose, 0.1 mM PMSF and 1 mM DTT. The resulting cells was suspended in 5 ml of the same buffer. Cells of *Flavobacterium indologenes* IFO 14944, *Lactobacillus brevis* IFO 13110, *Arthrobacter nicotianae* IFO 14234, *Bacillus subtilis* IFO 3007, *Klebsiella planticola* IFO 3317, *Escherichia coli* IFO 13168, *Pseudomonas putida* IFO 3738, *Stenotrophomonas maltophilia* IFO 12692, *Enterobacter cloacae* IFO 3320 or *Serratia marcescens* IFO 12648 were disrupted by passing through a French press homogenizer or by ultrasonic disintegration (Cosmo Bio Co., Ltd.) and others was treated with 2 mg lysozyme (Sigma) or 200 units zymolase (Sigma)/ml of cell suspension at 30°C for 1 hour before the disruption as shown in **Table 4**. The resulting homogenate was centrifuged to remove cell debris and the supernatant was dialyzed against 10 mM Tris-HCl (pH 7.5) buffer containing 15% sucrose and 0.1 mM PMSF and used as cell-free extract.

The enzyme reaction was carried out by incubating a tube with 500 µl of reaction mixture **A** comprising 2.5 mM DTP, 2.5 mM HT, 0.38 mM NADP⁺, 0.38 mM NAD⁺, 5 mM ATP, 193.25 µl cell-free extract and 80 mM Tris-HCl buffer, pH 7.50 or reaction mixture **B** supplemented with 8.4 mM MnCl₂ and 32 mM MgCl₂ at 28°C. After incubation for 2 hours, the reaction was slopped by heating in a boiling water bath for 3 minutes, the mixture was centrifuged at 10,000 x g for 10 minutes and the supernatant was treated with acid phosphatase at 37°C. After incubation for 30 minutes, vitamin B₆ produced in the reaction mixture was determined by the bioassay method using *Saccharomyces carlsbergensis* ATCC 9080. As a result, 7 - 23 and 33 - 139 ng of vitamin B₆/ml/mg protein/hour were produced in the reaction mixture **A** and **B**, respectively, as summarized in **Table 4**.

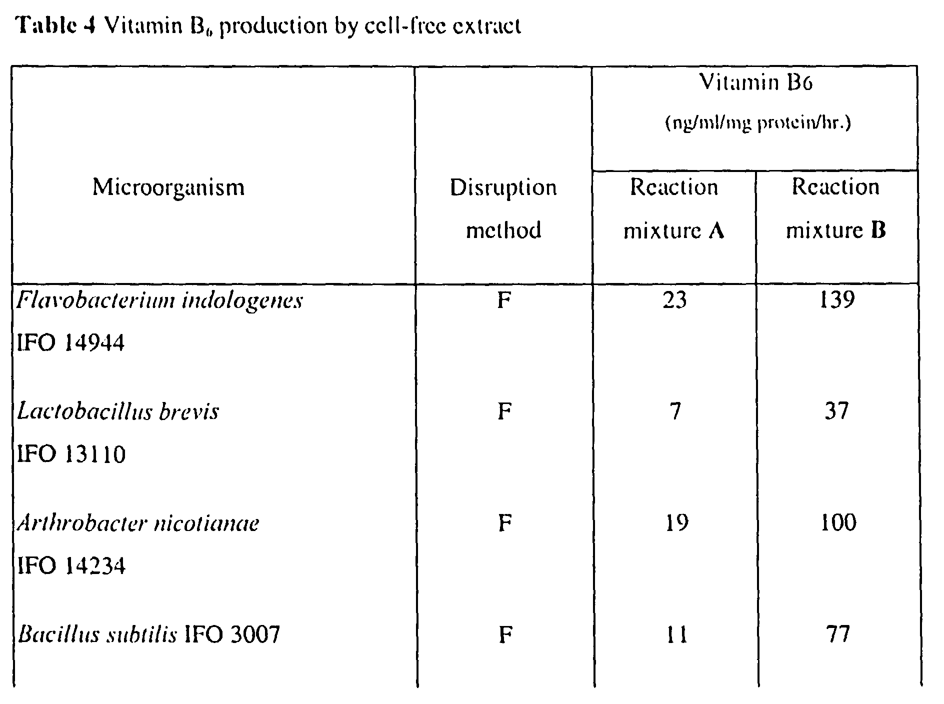

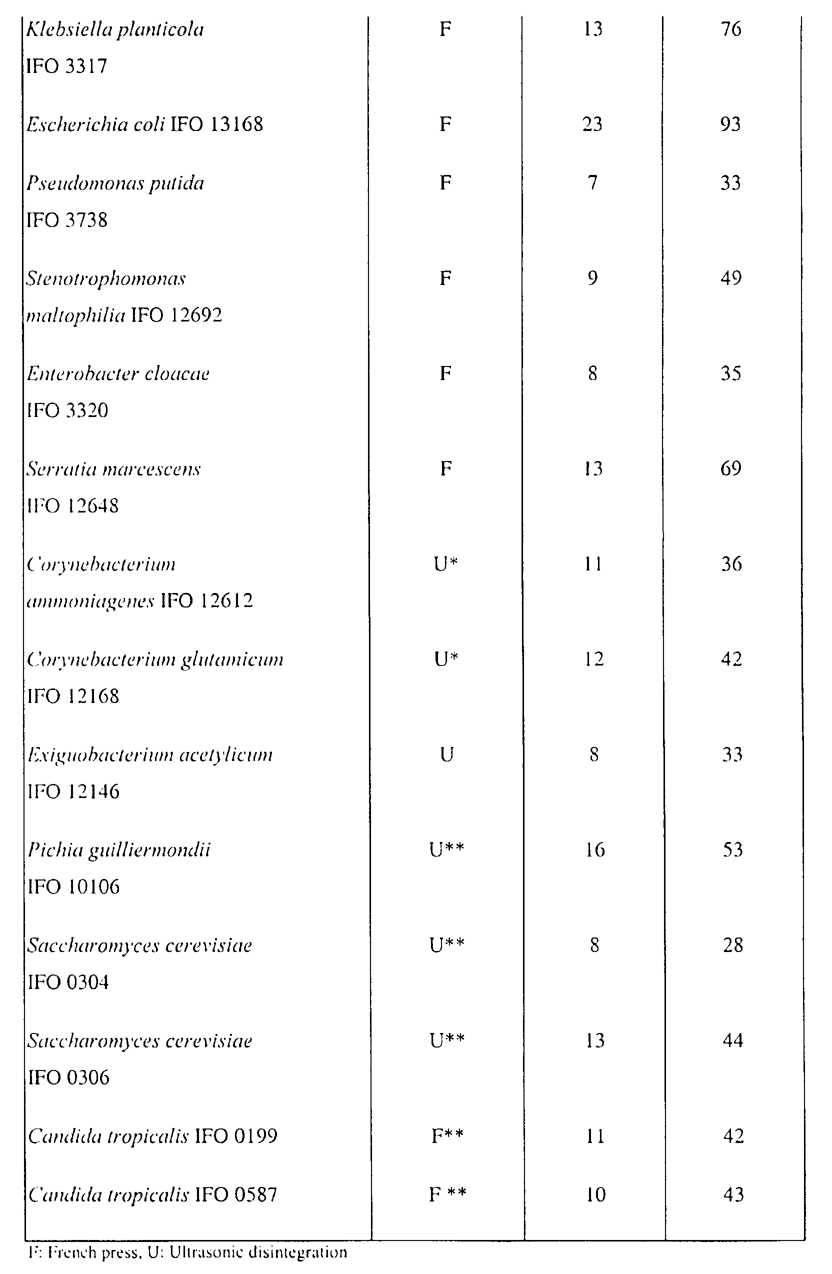

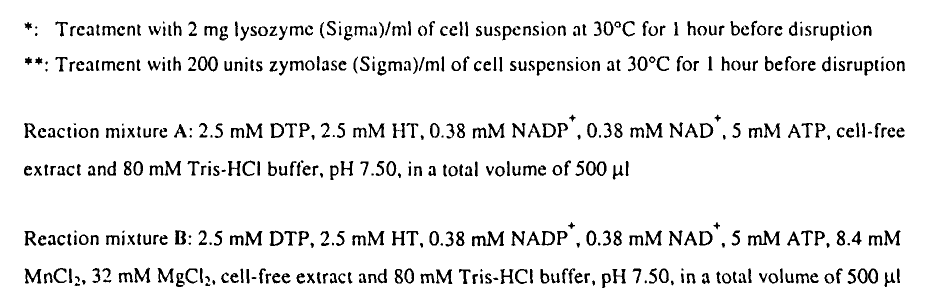

## Claims

1. A process for the production of vitamin B₆ from 1-deoxy-D-threo-pentulose (DTP) and 4-hydroxy-L-threonine (HT), which process comprises contacting DTP and HT with an enzyme reaction system prepared from cells of a microorganism capable of producing vitamin B₆ from DTP and HT, whereby said contacting occurs in the presence of nicotinamide adenine dinucleotide phosphate (NADP⁺), nicotinamide adenine dinucleotide (NAD⁺) and adenosine triphosphate (ATP).

2. A process according to claim 1, wherein the concentrations of DTP, HT, NADP⁺, NAD⁺ and ATP in the enzyme reaction system are 0.1mM or more, preferably from 1mM to 10mM for DTP and HT, preferably from 0.05 mM to 5 mM, more preferably from 0.2 mM to 0.4 mM, for NADP⁺ and NAD⁺, and preferably from 1 mM to 20 mM, more preferably from 3 mM to 7 mM, for ATP.

3. A process according to claim 1 or 2, wherein the enzyme reaction system contains in addition manganese ions, magnesium ions, or a mixture of both manganese and magnesium ions.

4. A process according to claim 3, wherein the concentrations of manganese ions and magnesium ions are in the case of manganese ions from 0.1 mM to 100 mM, preferably from 5 mM to 10 mM and in the case of magnesium ions from 3 mM to 300 mM, preferably from 20 mM to 50 mM.

5. A process according to any one of claims 1 to 4, wherein the enzyme reaction to prepare the enzyme reaction system is effected in a pH range from 6.0 to 8.5, preferably from 7.0 to 8.0, and in a temperature range from 15°C to 45°C, preferably from 20°C to 40°C, for 30 minutes to 5 hours, preferably for 2 hours to 4 hours.

6. A process according to any one of claims 1 to 5, wherein the enzyme reaction system contains the cell-free extract derived from a microorganism belonging to the genus *Rhizobium, Sinorhizobium, Flavobacterium, Chryseobacterium, Lactobacillus, Arthrobacter, Bacillus, Klebsiella, Escherichia, Pseudomonas, Stenotrophomonas, Enterobacter, Serratia, Corynebacterium, Brevibacterium, Exiguobacterium, Saccharomyces, Yamadazma, Pichia* or *Candida.*

7. A process according to claim 6, wherein the enzyme reaction system contains a cell-free extract derived from one or more of *Rhizobium meliloti* (also known as *Sinorhizobium meliloti*) IFO 14782 (DSM No. 10226), *Flavobacterium indologenes* (also known as *Chryseobacterium indologenes*) IFO 14944, *Lactobacillus brevis* IFO 13110, *Arthrobacter nicotianae* IFO 14234, *Bacillus subtilis* IFO 3007, *Klebsiella planticola* IFO 3317, *Escherichia coli* IFO 13168, *Pseudomonas putida* IFO 3738, *Stenotrophomonas maltophilia* (also known as *Pseudomonas maltophilia* or Xanthomonas *maltophilia*) IFO 12692, *Enterobacter cloacae* IFO 3320, *Serratia marcescens* IFO 12648, *Corynebacterium ammoniagenes* (also known as *Brevibacterium ammoniagenes*) IFO 12612, *Corynebacterium glutamicum* (also known as *Brevibacterium glutamicum*) IFO 12168, *Exiguobaterium acetylicum* (also known as *Brevibacterium acetylicum*) IFO 12146, *Pichia guilliermondii* (also known as *Yamadazyma guilliermondii*) IFO 10106, *Saccharmyces cerevisiae* IFO 0304 and IFO 0306 and *Candida tropicalis* IFO 0199 and IFO 0587.

8. A process according to claim 7, wherein the enzyme system contains a cell-free extract derived from one or more of *Rhizobium meliloti* IFO 14782 (DSM No. 10226), *Flavobacterium indologenes* IFO 14944, *Bacillus subtilis* IFO 3007, *Escherichia coli* IFO 13168, *Serratia marcescens* IFO 12648, *Corynebacterium ammoniagenes* IFO 12612, *Corynebacterium glutamicum* IFO 12168, *Pichia guilliermondii* IFO 10106 and *Saccharomyces cerevisiae* IFO 0306.
